# EUROPÄISCHE PATENTSCHRIFT

(11) **EP 1 191 890 B1**
(45) Veröffentlichungstag und Bekanntmachung des Hinweises auf die Patenterteilung: **16.03.2005**
(21) Anmeldenummer: 99926226.4
(22) Anmeldetag: 07.07.1999
(51) Int. Cl.: A61B 17/70

(54) **WINKELVERSTELLBARE KNOCHENSCHRAUBE UND VORRICHTUNG ZUR OSTEOSYNTHETISCHEN KNOCHENFIXATION**
ANGLE-ADJUSTABLE BONE SCREW AND DEVICE FOR THE OSTEOSYNTHETIC BONE FIXATION
VIS A OS A AJUSTAGE ANGULAIRE ET DISPOSITIF DE FIXATION POUR OS EN OS DE SYNTHESE

(43) Veröffentlichungstag der Anmeldung: 03.04.2002
(73) Patentinhaber: SYNTHES AG Chur, 7002 Chur (CH)
(72) Erfinder: FRIGG, Robert, CH-2544 Bettlach (CH); FERUS, Robert, CH-4574 Nennigkofen (CH)
(74) Vertreter: Lusuardi, Werther Giovanni, Dr.
(86) Internationale Anmeldenummer: PCT/CH1999/000302
(87) Internationale Veröffentlichungsnummer: WO 2001/003591

(56) Entgegenhaltungen:
- EP-A- 0 498 709
- EP-A- 0 507 162
- WO-A-98/34553
- WO-A-98/41160
- WO-A-99/03415
- US-A- 5 466 237

## Beschreibung

Die Erfindung bezieht sich auf eine Knochenschraube gemäss dem Oberbegriff des Patentanspruchs 1. Eine Knockenschraube gemäß dem Oberbegriff des Anspruchs 1 ist aus WO-A-98/34553 bekannt.

Aus dem Stand der Technik sind bereits verschiedene Vorrichtung zur internen Fixation von Knochenfragmenten im menschlichen oder tierischen Körper bekannt.

im Falle der internen Fixation der Wirbelsäule oder von Wirbelsäulenteilen bestehen solche Vorrichtungen oft im wesentlichen aus Pedikelschrauben, welche in den Pedikeln der einzelnen zu verbindenden Wirbelkörper mittels Gewinden verankert werden, und einem oder mehreren sich in Richtung der Wirbelsäule erstreckenden Längsträger, welcher fest mit der Pedikelschraube verbunden sein muss. Zur stabilen Verankerung des gesamten Implantates müssen die Pedikelschrauben einerseits fest in die Pedikel eingeschraubt und andererseits starr mit den Längsträgern verbunden werden. Die Verbindung zwischen dem Schraubenkopf der Pedikelschrauben und dem Längsträger erfolgt üblicherweise mittels Klemmmechanismen, welche auch bei verschiedenen Winkeln der Pedikelschraube gegenüber dem Längsträger eine stabile Verbindung ermöglichen müssen. Die Klemmverbindung muss lösbar sein, damit das gesamte Implantat ohne grosse Gewebeöffnungen im Bereich der Wirbelsäule wieder entfernbar ist.

Feste Verbindungen zwischen Knochenschrauben und Platten oder Trägem sind auch bei anderen internen Knochenfixationen häufig. Auch hier müssen verschiedene Abwinklung der Knochenschrauben gegenüber der Platte oder dem Träger möglich sein, ohne dass die Verbindungen in ihrer Stabilität beeinträchtigt werden.

Eine solche Verbindung zwischen einer Knochenverankerungsschraube und einem Stabilisationsstab zur internen Fixation von Wirbelkörpern ist aus der US 5,466,237 BYRD bekannt. Diese bekannte Erfindung weist eine Knochenverankerungsschraube mit einem Schraubenkopf auf, welcher an seiner dem Schraubenschaft zugewandten Seite kugelschichtförmig gestaltet ist und endständig konvex ausgebildet ist. Der kugelschichtförmige Teil der Schraube ist in einer Bohrung des Verankerungselementes gelagert, wobei diese Bohrung einen konkaven sich gegen den Schraubenschaft verjüngenden Abschnitt umfasst, so dass sich eine kugelgelenkartige Verbindung zwischen der Knochenschraube und dem Verankerungselement ergibt. Blockiert wird diese kugelgelenkartige Verbindung durch Anziehen einer Mutter am Verankerungselement, welche auf den in das Verankerungselement eingelegten Längsträger presst, welcher in der Folge auf den endständigen, konvexen Teil des Schraubenkopfes drückt und somit den Schraubenkopf im Verankerungselement blockiert. Eine solche blockierbare kugelgelenkartige Verbindung ist jedoch wegen der häufig nicht exakt passenden sphärischen Flächen an der Knochenschraube und im Verankerungselement für die Aufnahme der bei der Knochenfixation auftretenden Kräfte ungeeignet. Zudem ist eine solche kugelgelenkartige Verbindung nur kraftschlüssig blockierbar.

Hier will die Erfindung Abhilfe schaffen. Der Erfindung liegt die Aufgabe zugrunde, eine Verbindung zwischen Knochenschraube und Verankerungselement herzustellen, welche verschiedene Winkel zwischen Schraubenachse und Verankerungselement zulässt und stabil, insbesondere bei geeigneter Materialpaarung formschlüssig ist.

Die Erfindung löst die gestellte Aufgabe mit einer Knochenschraube, welche die Merkmale des Anspruchs 1 aufweist, sowie mit einer Vorrichtung zur osteosynthetischen Knochenfixation, welche die Merkmale des Anspruchs 23 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Gestaltung der Bohrung zur Aufnahme einer Knochenschraube und der Gestaltung der Knochenschraube mit einem Wulst, welcher zur Auflage in einem konkaven Abschnitt der Bohrung bestimmt ist, ein linienförmiger Kontakt herstellbar ist, welcher bei der Fixation der Vorrichtung zu einer festen Verbindung zwischen Knochenschraube und Fixationskörper führt. Im Falle einer deformierbaren Bohrungswand lässt sich wegen des linienförmigen Kontakts auch eine formschlüssige Verbindung zwischen Wulst und Bohrungswand herstellen. Die durch die Zweiteiligkeit der Knochenschraube erreichbaren Vorteile liegen darin, dass die Oberfläche des konvexen Schraubenkopfes im Bereich der Berührung mit einem anderen implantatteil, beispielsweise dem Längsträger glatt ist und diese Kontaktzone nicht durch Mittel zur Aufnahme eines Schraubendrehers beeinträchtigt wird.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Schnitt durch eine Vorrichtung;
Fig. 2 einen Schnitt parallel zu einem Längsträger durch eine weitere Vorrichtung;
Fig. 3 einen Schnitt durch die in Fig. 2 dargestellte Vorrichtung quer zu einem Längsträger;
Fig. 4 eine Ansicht einer mehrteiligen Knochenschraube;
Fig. 5 eine Ansicht einer weiteren mehrteiligen Knochenschraube;
Fig. 6 eine Ansicht nochmals einer weiteren mehrteiligen Knochenschraube;
Fig. 7 einen Schnitt durch eine weitere Vorrichtung; und
Fig. 8 eine Ansicht einer Ausführungsform der erfindungsgemässen Knochenschraube.

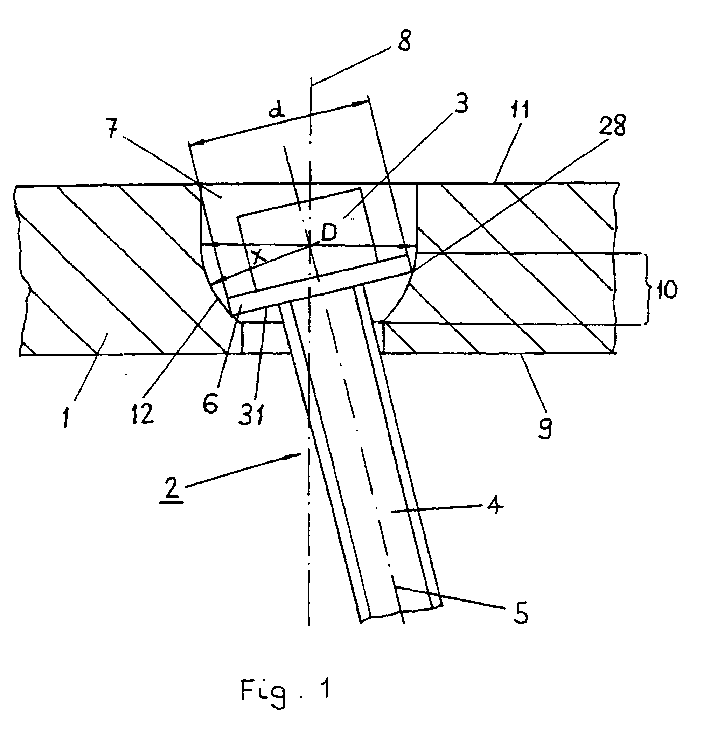

In Fig. 1 ist ein Ausschnitt aus einem Knochenfixationskörper 1 zusammen mit einer Knochenschraube 2 dargestellt. Der Knochenfixationskörper 1 ist als Knochenplatte gestaltet und weist eine schraubenschaftseitige Unterseite 9 und eine schraubenkopfseitige Oberseite 11 auf, wobei die Unterseite 9 beim Festschrauben der Knochenplatte zur Anlage an den Knochen bestimmt ist. Die Aufnahme der Knochenschraube 2 im Knochenfixationskörper 1 erfolgt in einer Bohrung 7, welche eine Zentralachse 8 aufweist, den Knochenfixationskörper 1 durchdringt und mit einem konkaven sich gegen die Unterseite 9 verjüngenden Abschnitt 10 versehen ist. Der konkave Abschnitt 10 ist sphärisch mit einem Krümmungsradius X ausgebildet und mündet gegen die Oberseite 11 in einen zylindrischen Abschnitt mit dem Durchmesser D. In der hier gezeigten Ausführungsform der erfindungsgemässen Vorrichtung entspricht der Krümmungsradius X dem Radius des zylindrischen Abschnittes X = D / 2. Die Knochenschraube 2 umfasst konzentrisch zu einer Schraubenlängsachse 5 einen im Knochen oder Knochenteil zu verankernden Schraubenschaft 4 und einen Schraubenkopf 3 sowie zwischen Schraubenschaft 4 und Schraubenkopf 3 einen zur Schraubenlängsachse 5 konzentrischen kreisscheibenförmigen Wulst 6 mit einer ebenen schraubenschaftseitigen Auflagefläche 31.
Der Wulst 6 ist bezüglich seines Durchmessers d so dimensioniert, dass er im konkaven Abschnitt 10 unter verschiedenen Winkeln zwischen der Schraubenlängsachse 5 und der Zentralachse 8 der Bohrung 7 an der Wand 12 der Bohrung 7 aufliegen kann. Damit ist gewährleistet, dass die Knochenschraube 2 unter verschiedenen Winkeln gegenüber dem Knochenfixationskörper 1 in den Knochen oder das Knochenteil schraubbar ist.

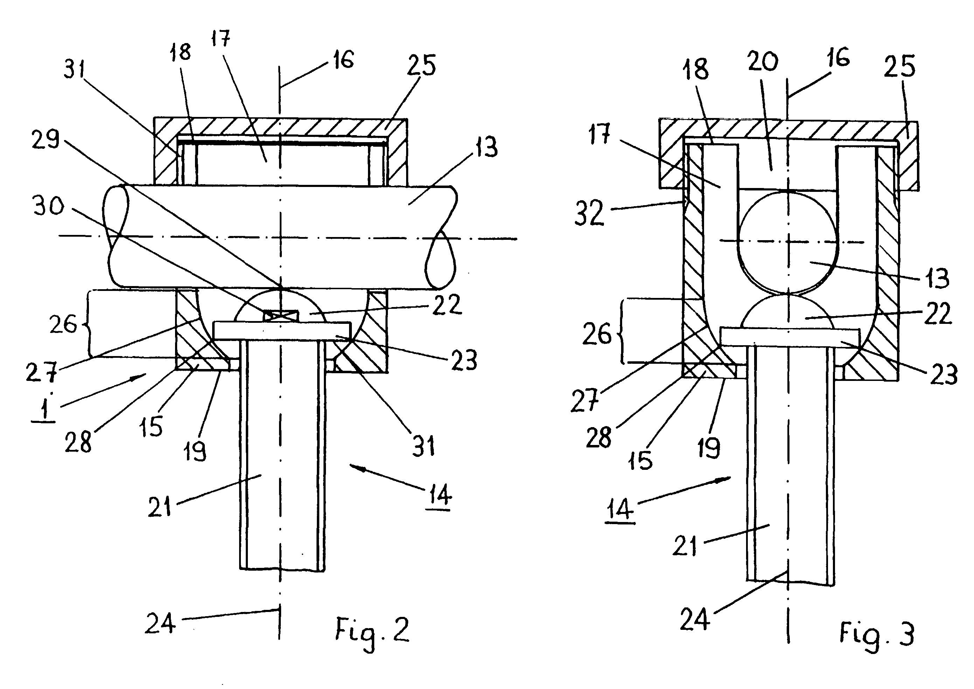

Die Fig. 2 und 3 zeigen eine Vorrichtung, welche zur Verbindung eines Längsträgers 13 mit einer Pedikelschraube 14 innerhalb eines Wirbelsäulenfixationssystems dient. Diese Vorrichtung umfasst eine Pedikelschraube 14, welche konzentrisch zu ihrer Schraubenlängsachse 24 einen im Knochen zu verankernden Schraubenschaft 21 und einen konvexen Schraubenkopf 22, einen Aufnahmekopf 15 mit der Zentralachse 16, welcher zur Verbindung eines Längsträgers 13 mit der Pedikelschraube 14 dient, und Spannmittel 25, welche im wesentlich die Form einer Mutter aufweisen und mittels eines Innengewindes 32 über ein an die Oberseite 18 des Aufnahmekopfes 15 angrenzendes Aussengewinde 31 in lösbarer Weise schraubbar sind und zur Fixierung des Längsträgers 13 und der Pedikelschraube 14 innerhalb des Aufnahmekopfes 15 dienen.

Der konvexe Schraubenkopf 22 ist kugelsegmentförmig ausgeführt, wobei der Zenit 29 des Kugelsegmentes auf der Schraubenlängsachse 24 liegt und das schraubenkopfseitige Ende der Pedikelschraube 14 bildet. Zudem sind am Schraubenkopf 22 zwei oder mehr parallel zur Schraubenlängsachse 24 ausgerichtete Flächen 30 als Aussenzweikant zum Eindrehen der Pedikelschraube 14 in den Knochen mittels eines Schraubendrehers angebracht. Anstelle des Aussenzweikants ist auch ein Aussensechskant möglich.

Der Aufnahmekopf 15 weist eine schraubenkopfseitige Oberseite 18, eine schraubenschaftseitige Unterseite 19, eine koaxial zur Zentralachse 16 den Aufnahmekopf 15 durchdringende Durchgangsbohrung 17 zur Aufnahme der Pedikelschraube 14 und zusätzlich einen quer zur Zentralachse 16 verlaufenden gegen die Oberseite 18 offenen Kanal 20 zur Aufnahme eines Längsträgers 13 auf. Auf diese Weise ist der Längsträger 13 von der Oberseite 18 her in den offenen Kanal 20 einlegbar und dort mittels des Spannmittels 25 in lösbarer Weise fixierbar.

Die Durchgangsbohrung 17 umfasst einen konkaven sich gegen die Unterseite 19 verjüngenden Abschnitt 26, welcher in der hier dargestellten Vorrichtung kugelschichtartig ausgestaltet ist.

Zudem weist die Pedikelschraube 14 zwischen dem konvexen Schraubenkopf 22 und dem Schraubenschaft 21 einen zur Schraubenlängsachse 24 konzentrischen scheibenförmigen Wulst 23 auf, welcher so dimensioniert ist, dass der Wulst 23 im konkaven Abschnitt 26 der Durchgangsbohrung 17 unter verschiedenen Winkeln zwischen Schraubenlängsachse 24 und Zentralachse 16 an der Wand 27 der Durchgangsbohrung 17 auflegbar ist.

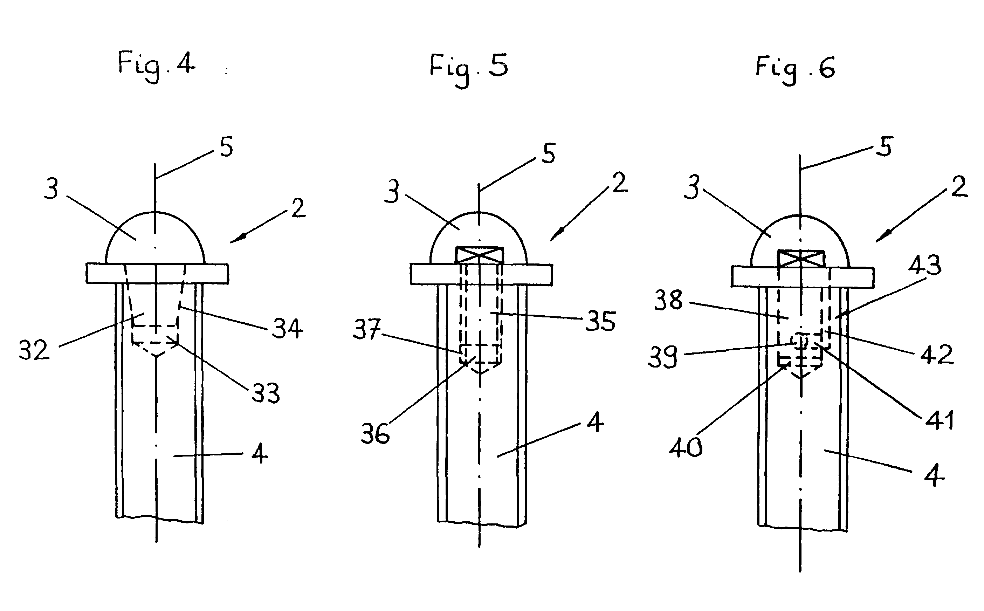

In Fig. 4 ist eine zweiteilige Knochenschraube 2 dargestellt. Die Verbindung zwischen Schraubenkopf 3 und Schraubenschaft 4 ist eine Konusverbindung. Am Schraubenkopf 3 ist konzentrisch zur Schraubenlängsachse 5 ein konischer Zapfen 32 angebracht, welcher in einer zur Schraubenlängsachse 5 konzentrische Bohrung 33 mit einem innenkonus 34 am schraubenkopfseitigen Ende des Schraubenschaftes 4 befestigbar ist.

Fig. 5 zeigt eine weitere zweiteilige Knochenschraube 2. Hier ist die Verbindung zwischen Schraubenkopf 3 und Schraubenschaft 4 eine Schraubverbindung. Am Schraubenkopf 3 ist konzentrisch zur Schraubenlängsachse 5 ein Gewindezapfen 35 angebracht, welcher in eine zur Schraubenlängsachse 5 konzentrische Bohrung 36 mit einem Innengewinde 37 am schraubenkopfseitigen Ende des Schraubenschaftes 4 schraubbar ist.

Fig. 6 zeigt wieder eine weitere zweiteilige Knochenschraube 2. Hier ist die Verbindung zwischen Schraubenkopf 3 und Schraubenschaft 4 ein Bajonettverschluss. Am Schraubenkopf 3 ist konzentrisch zur Schraubenlängsachse 5 ein Zapfen 38 mit einem radial vorstehenden Stift 39 angebracht, welcher in eine zur Schraubenlängsachse 5 konzentrische Bohrung 40 mit einer Nute 43 einschnappbar ist, wobei die Nute 43 einen parallel zur Schraubenlängsachse 5 verlaufenden Teil 42 und einer peripher in der Bohrung 40 verlaufenden Teil 41 aufweist.

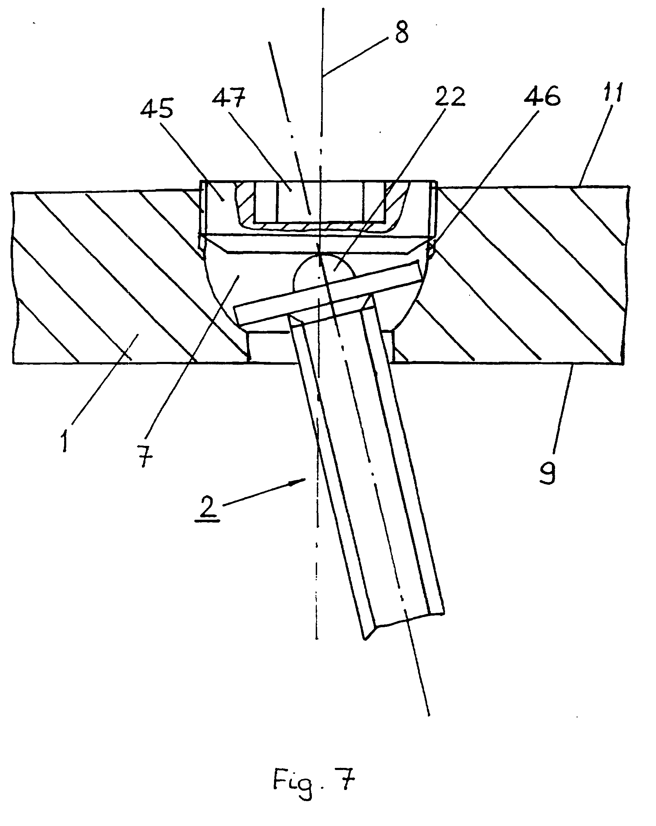

Fig. 7 zeigt eine Vorrichtung, welche sich nur darin von der in Fig. 1 dargestellten Ausführungsform unterscheidet, dass der Fixationskörper 1 eine Knochenplatte mit mindestens einer durchgehenden Bohrung 7 für eine Knochenschraube 2 ist und die Vorrichtung zusätzlich eine Madenschraube 45 mit Mitteln 47 zur Aufnahme eines Schraubendrehers umfasst, welche in ein von der Oberseite 11 her in die Bohrung 7 eingebrachtes Innengewinde 46 schraubbar ist und beim Anziehen gegen den Schraubenkopf 22 pressbar ist. Mittels dieser festziehbaren Madenschraube 45 wird eine winkelstabile Fixation der Knochenschraube 2 in der Knochenplatte 1 erreicht.

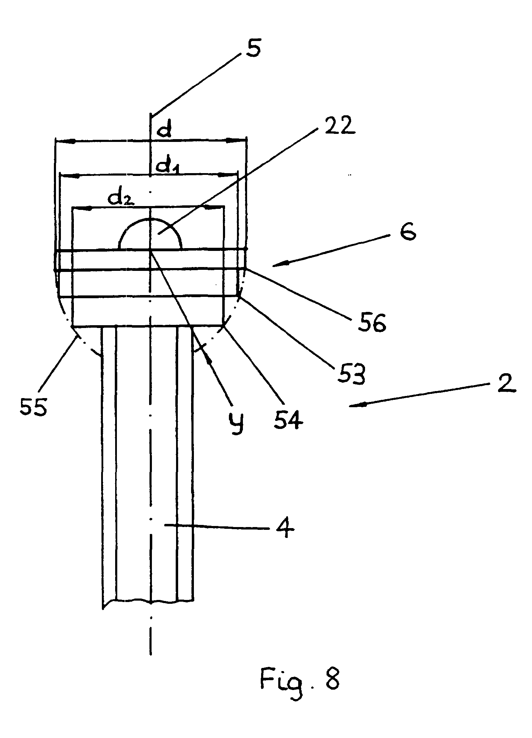

In Fig. 8 ist eine erfindungsgemässe Knochenschraube 2 dargestellt, weiche sich nur dadurch von den in den Fig. 4 bis 6 dargestellten Ausführungsformen unterscheidet, dass der Wulst 6 schraubenschaftseitig mehrere kreislinienförmige Kanten 53;54;56 umfasst, wobei die Durchmesser d;d₁;d₂ dieser Kanten 53;54;56 so dimensioniert sind, dass die Kanten 53;54;56 auf einer imaginären, schraubenschaftseitig konvexen Fläche 55, welche als zur Zentralachse 5 konzentrische Kugelzone mit dem Radius Y ausgebildet ist, verlaufen.

## Patentansprüche

1. Knochenschraube (2;14) mit einem zu einer Schraubenlängsachse (5;24) konzentrisch in einem Knochen oder Knochenteil zu verankernden Schraubenschaft (4;21) und einem Schraubenkopf (3;22), wobei die Knochenschraube (2;14) zwischen dem Schraubenkopf (3;22) und dem Schraubenschaft (4;21) einen zur Schraubenlängsachse (5;24) konzentrischen scheibenförmigen Wulst (6;23) aufweist, dessen Durchmesser grösser als der Durchmesser des Schraubenschaftes (4;21) ist, wobei
der Wulst (6;23) mehrere, zur Schraubenlängsachse (5;24) konzentrische Kanten (53;54;56) umfasst,
**dadurch gekennzeichnet, dass**
A) die Kanten (53;54;56) kreislinienförmig ausgebildet sind und die Durchmesser d;d₁;d₂;dᵢ aufweisen, weiche so dimensioniert sind, dass die Kanten (53;54;56) auf einer imaginären, schraubenschaftseitig konvexen Fläche (55) verlaufen, und
B) die imaginäre Fläche (55) eine zur Zentralachse (8;16) konzentrische Kugelzone mit dem Radius Y ist.

2. Knochenschraube (2;14) nach Anspruch 1, **dadurch gekennzeichnet, dass** der Wulst (6;23) zwei, zur Schraubenlängsachse (5;24) konzentrische Kanten (53;54;56) aufweist.

3. Knochenschraube (2;14) nach Anspruch 2, **dadurch gekennzeichnet, dass** die konzentrischen Kanten (53;54;56) des Wulstes (6;23) gegen den Schraubenschaft (4) hin abnehmende Durchmesser d > d₁ > d₂ > dᵢ aufweisen.

4. Knochenschraube (2) nach einem der Ansprüche 1 bis 3, **dadurch gekennzeichnet, dass** der Schraubenkopf (22) konvex ausgebildet ist.

5. Knochenschraube (2) nach Anspruch 4, **dadurch gekennzeichnet, dass** der Schraubenkopf (22) sphärisch ausgebildet ist.

6. Knochenschraube (2) nach Anspruch 5, **dadurch gekennzeichnet, dass** der Schraubenkopf (22) halbkugelförmig ausgebildet ist, wobei der Zenit des Schraubenkopfes (22) die Schraubenlängsachse (24) endständig schneidet.

7. Knochenschraube (2) nach einem der Ansprüche 1 bis 6, **dadurch gekennzeichnet, dass** der Schraubenkopf (3;22) einstückig mit dem Schraubenschaft (4;21) ist.

8. Knochenschraube (2) nach einem der Ansprüche 1 bis 6, **dadurch gekennzeichnet, dass** sie mehrteilig ist, wobei mindestens Schraubenkopf (3;22) und Schraubenschaft (4;21) separate aber konzentrisch zur Schraubenlängsachse (5;24) verbindbare Einzelteile sind.

9. Knochenschraube (2) nach Anspruch 8, **dadurch gekennzeichnet, dass** der Schraubenkopf (3;22) und der Schraubenschaft (4;21) lösbar verbindbare Einzelteile sind.

10. Knochenschraube (2) nach Anspruch 8, **dadurch gekennzeichnet, dass** der Schraubenkopf (3;22), der Wulst (6;23) und der Schraubenschaft (4;21) separate aber konzentrisch zur Schraubenlängsachse (5;24) verbindbare Einzelteile sind.

11. Knochenschraube (2) nach einem der Ansprüche 8 bis 10, **dadurch gekennzeichnet, dass** der Schraubenkopf (3;22) mittels einer Konusverbindung mit dem Schraubenschaft (4;21) verbunden ist.

12. Knochenschraube (2) nach einem der Ansprüche 8 bis 10, **dadurch gekennzeichnet, dass** der Schraubenkopf (3;22) mittels einer Schraubverbindung mit dem Schraubenschaft (4;21) verbunden ist.

13. Knochenschraube (2) nach einem der Ansprüche 8 bis 10, **dadurch gekennzeichnet, dass** der Schraubenkopf (3;22) mittels eines Bajonettverschlusses mit dem Schraubenschaft (4;21) verbunden ist.

14. Knochenschraube (2) nach einem der Ansprüche 1 bis 13, **dadurch gekennzeichnet, dass** der Durchmesser d des Wulstes (6;23) zwischen 8-10 mm beträgt.

15. Knochenschraube (2) nach einem der Ansprüche 1 bis 16, **dadurch gekennzeichnet, dass** der Wulst (6;23) eine Dicke von 1-2 mm hat.

16. Knochenschraube (2) nach einem der Ansprüche 1 bis 17, **dadurch gekennzeichnet, dass** der Aussendurchmesser des Schraubenschaftes (4;21) 5-6 mm beträgt.

17. Knochenschraube (2) nach einem der Ansprüche 1 bis 15, **dadurch gekennzeichnet, dass** der Durchmesser d des Wulstes (6;23) zwischen 4-6 mm beträgt.

18. Knochenschraube (2) nach Anspruch 17, **dadurch gekennzeichnet, dass** der Wulst (6;23) eine Dicke von 0,5 - 1 mm hat.

19. Knochenschraube (2) nach Anspruch 18, **dadurch gekennzeichnet, dass** der Aussendurchmesser des Schraubenschaftes (4;21) 3-5 mm beträgt.

20. Knochenschraube (2) nach einem der Ansprüche 1 bis 19, **dadurch gekennzeichnet, dass** sie zur Fixation von Knochen oder Knochenteilen innerhalb einer osteosynthetischen Fixationsvorrichtung dient.

21. Knochenschraube (2) nach Anspruch 20, **dadurch gekennzeichnet, dass** sie zur Fixation von Knochen oder Knochenteilen an einer Knochenplatte (1) dient.

22. Knochenschraube (2) nach Anspruch 20, **dadurch gekennzeichnet, dass** sie eine Pedikelschraube (14) ist und zur Fixation von Wirbelkörpern innerhalb einer Wirbelsäulenfixationsvorrichtung dient.

23. Vorrichtung zur osteosynthetischen Knochenfixation mit mindestens einer Knochenschraube (2;14) nach einem der Ansprüche 1 bis 22 ; und
A) mindestens einem plattenförmigen, prismatischen oder zylindrischen Fixationskörper (1;15), weicher mindestens eine Bohrung (7;17) mit einer Zentralachse (8;16) zur Aufnahme der Knochenschraube (2;14), schraubenschaftseitig eine Unterseite (9;19) und schraubenkopfseitig eine Oberseite (11;18) aufweist, wobei
B) die Bohrung (7;17) einen sich gegen die Unterseite (9;19) verjüngenden Abschnitt (10;26) umfasst,
**dadurch gekennzeichnet, dass**
C) der Durchmesser d des Wulstes (6;23) so dimensioniert ist, dass der Wulst (6;23) im konkaven Abschnitt (10;26) der Bohrung (7;17) unter verschiedenen Winkeln zwischen Schraubenlängsachse (5;24) und Zentralachse (8;16) an der Wand (12;27) der Bohrung (7;17) zur Anlage bringbar ist.

24. Vorrichtung nach Anspruch 23, **dadurch gekennzeichnet, dass**
A) die Knochenschraube (2) eine Pedikelschraube (14) mit einem konvexen Schraubenkopf (22) ist;
B) der Fixationskörper (1) ein Aufnahmekopf (15) mit der Zentralachse (16) ist, welcher zur Verbindung eines Längsträgers (13) mit der Pedikelschraube (14) dient, und dieser Aufnahmekopf (15) zusätzlich einen quer zur Zentralachse (16) verlaufenden, gegen die Oberseite (18) offenen Kanal (20) zur Aufnahme eines Längsträgers (13) aufweist; und
C) die Vorrichtung zusätzlich Spannmittel (25) umfasst, welche von der Oberseite (18) her mit dem Aufnahmekopf (15) in lösbarer Weise verbindbar sind und zur Fixierung eines Längsträgers (13) und der Pedikelschraube (14) innerhalb des Aufnahmekopfes (15) dienen.

25. Vorrichtung nach Anspruch 23 oder 24, **dadurch gekennzeichnet, dass** der kreisscheibenförmige Wulst (6;23) den Durchmesser d aufweist und der konkave Abschnitt (10;26) sphärisch ausgebildet ist und den Durchmesser D aufweist, wobei D = d ist.

26. Vorrichtung nach Anspruch 23 oder 24, **dadurch gekennzeichnet, dass** der kreisscheibenförmige Wulst (6;23) den Durchmesser d aufweist und der konkave Abschnitt (10;26) sphärisch ausgebildet ist und den Durchmesser D aufweist, wobei D > d ist.

27. Vorrichtung nach Anspruch 26, **dadurch gekennzeichnet, dass** das Verhältnis d : D zwischen 0,5 und 1,0 liegt.

28. Vorrichtung nach Anspruch 27, **dadurch gekennzeichnet, dass** das Verhältnis d : D zwischen 0,85 und 0,95 liegt.

29. Vorrichtung nach Anspruch 23 oder 24, **dadurch gekennzeichnet, dass** der konkave Abschnitt (10;26) kugelschichtartig ausgebildet ist, wobei die Kugelschicht einen Radius X aufweist und X ≥ D/2 ist.

30. Vorrichtung nach Anspruch 29, **dadurch gekennzeichnet, dass** das Verhältnis von D/2 zu X zwischen 0,5 und 1,0 beträgt.

31. Vorrichtung nach Anspruch 30, **dadurch gekennzeichnet, dass** das Verhältnis von D/2 zu X zwischen 0,85 und 0,95 beträgt.

32. Vorrichtung nach einem der Ansprüche 23 bis 31, **dadurch gekennzeichnet, dass** der Rand des Wulstes (6;23) kantig ausgebildet ist und mindestens eine untere Kante (28;53;54;56) aufweist.

33. Vorrichtung nach einem der Ansprüche 23 bis 33, **dadurch gekennzeichnet, dass** der Wulst (6) mehrere, zur Schraubenlängsachse (5;24) konzentrische Kanten (53;54;56) mit gegen den Schraubenschaft (4) hin abnehmenden Durchmessern d > d₁ > d₂ umfasst.

34. Vorrichtung nach Anspruch 33, **dadurch gekennzeichnet, dass** die Kanten (53;54;56) kreislinienförmig sind, und die Durchmesser d;d₁;d₂ so dimensioniert sind, dass die Kanten (53;54;56) auf einer imaginären, schraubenschaftseitig konvexen Fläche (55) verlaufen und im konkaven Abschnitt (10;26) der Bohrung (7;17) unter verschiedenen Winkeln zwischen Schraubenlängsachse (5;24) und Zentralachse (8;16) an der Wand (12;27) der Bohrung (7; 17) zur Anlage bringbar sind.

35. Vorrichtung nach Anspruch 34, **dadurch gekennzeichnet, dass** die imaginäre Fläche (55) eine zur Zentralachse (8;16) konzentrische Kugelzone mit dem Radius Y ist.

36. Vorrichtung nach einem der Ansprüche 23 oder 25 bis 35, **dadurch gekennzeichnet, dass** der Fixationskörper (1) eine Knochenplatte mit mindestens einer durchgehenden Bohrung (7) für eine Knochenschraube (2) ist und die Vorrichtung zusätzlich eine Madenschraube (45) mit Mitteln zur Aufnahme eines Schraubendrehers umfasst, welche in ein von der Oberseite (11) her in die Bohrung (7) eingebrachtes Innengewinde (46) schraubbar ist und beim Anziehen gegen den Schraubenkopf (22) pressbar ist.

## Claims

1. A bone screw (2; 14) with a screw shank (4; 21) to be anchored concentric to a longitudinal axis (5; 24) of the screw in a bone or bone part, and with a screw head (3; 22), whereby the bone screw (2; 14) has, between the screw head (3; 22) and the screw shank (4; 21), a disk-shaped collar (6; 23) which is concentric to the longitudinal axis (5; 24) of the screw and whose diameter is greater than the diameter of the screw shank (4; 21), whereby
the collar (6; 23) comprises several edges (53; 54; 56) concentric to the longitudinal axis (5; 24) of the screw,
**characterized in that**
A) the edges (53; 54; 56) are circular, and the diameters d; d₁; d₂; dᵢ are dimensioned such that the edges (53; 54; 56) run on an imaginary convex surface (55) on the screw shank side; and
B) the imaginary surface (55) is a spherical zone concentric to the central axis (8; 16) and with the radius Y.

2. The bone screw (2; 14) as claimed in claim 1, **characterized in that** the collar (6; 23) has two edges (53; 54; 56) concentric to the longitudinal axis (5; 24) of the screw.

3. The bone screw (2; 14) as claimed in claim 2, **characterized in that** the concentric edges (53; 54; 56) of the collar (6; 23) have diameters d > d₁ > d₂ decreasing toward the screw shank (4).

4. The bone screw (2) as claimed in one of claims 1 through 3, **characterized in that** the screw head (22) is of convex design.

5. The bone screw (2) as claimed in claim 4, **characterized in that** the screw head (22) is of spherical design.

6. The bone screw (2) as claimed in claim 5, **characterized in that** the screw head (22) is of semispherical design, the zenith of the screw head (22) intersecting the longitudinal axis (24) of the screw at the end.

7. The bone screw (2) as claimed in one of claims 1 through 6, **characterized in that** the screw head (3; 22) is made in one piece with the screw shank (4; 21).

8. The bone screw (2) as claimed in one of claims 1 through 6, **characterized in that** it is in more than one piece, at least screw head (3; 22) and screw shank (4; 21) being individual parts which are separate but can be connected concentric to the longitudinal axis (5; 24) of the screw.

9. The bone screw (2) as claimed in claim 8, **characterized in that** the screw head (3; 22) and the screw shank (4; 21) are individual parts which can be connected in a releasable manner.

10. The bone screw (2) as claimed in claim 8, **characterized in that** the screw head (3; 22), the collar (6; 23) and the screw shank (4; 21) are individual parts which are separate but can be connected concentric to the longitudinal axis (5; 24) of the screw.

11. The bone screw (2) as claimed in one of claims 8 through 10, **characterized in that** the screw head (3; 22) is connected to the screw shank (4; 21) by means of a cone connection.

12. The bone screw (2) as claimed in one of claims 8 through 10, **characterized in that** the screw head (3; 22) is connected to the screw shank (4; 21) by means of a screw connection.

13. The bone screw (2) as claimed in one of claims 8 through 10, **characterized in that** the screw head (3; 22) is connected to the screw shank (4; 21) by means of a bayonet lock.

14. The bone screw (2) as claimed in one of claims 1 through 13, **characterized in that** the diameter d of the collar (6; 23) is between 8 and 10 mm.

15. The bone screw (2) as claimed in one of claims 1 through 14, **characterized in that** the collar (6; 23) has a thickness of 1 to 2 mm.

16. The bone screw (2) as claimed in one of claims 1 through 15, **characterized in that** the external diameter of the screw shank (4; 21) is 5 to 6 mm.

17. The bone screw (2) as claimed in one of claims 1 through 15, **characterized in that** the diameter d of the collar (6; 23) is between 4 and 6 mm.

18. The bone screw (2) as claimed in claim 17, **characterized in that** the collar (6; 23) has a thickness of 0.5 to 1 mm.

19. The bone screw (2) as claimed in claim 18, **characterized in that** the external diameter of the screw shank (4; 21) is 3 to 5 mm.

20. The bone screw (2) as claimed in one of claims 1 through 19, **characterized in that** it serves for the fixation of bones or bone parts in an osteosynthesis fixation device.

21. The bone screw (2) as claimed in claim 20, **characterized in that** it serves for the fixation of bones or bone parts on a bone plate (1).

22. The bone screw (2) as claimed in claim 20, **characterized in that** it is a pedicle screw (14) and serves for the fixation of vertebrae in a spinal column fixation device.

23. A device for osteosynthetic bone fixation with at least one bone screw (2; 14) as claimed in one of claims 1 through 22; and
A) at least one plate-shaped, prismatic or cylindrical fixation body (1; 15) which has at least one bore (7; 17) with a central axis (8; 16) for receiving the bone screw (2; 14), an underside (9; 19) toward the screw shank, and an upper side (11; 18) toward the screw head,
B) the bore (7; 17) comprising a portion (10; 26) tapering toward the underside (9; 19),
**characterized in that**
C) the diameter d of the collar (6; 23) is dimensioned such that the collar (6; 23), in the concave portion (10; 26) of the bore (7; 17), can be made to bear on the wall (12; 27) of the bore (7; 17) at different angles between the longitudinal axis (5; 24) of the screw and the central axis (8; 16).

24. The device as claimed in claim 23, **characterized in that**
A) the bone screw (2) is a pedicle screw (14) with a convex screw head (22);
B) the fixation body (1) is a receiving head (15) with central axis (16), which serves to connect a longitudinal support (13) to the pedicle screw (14), and this receiving head (15) additionally has a channel (20) extending transverse to the central axis (16) and open toward the upper side (18) in order to receive a longitudinal support (13); and
C) the device additionally has clamping means (25) which can be connected to the receiving head (15) in a releasable manner from the upper side (18) and serve for fixing a longitudinal support (13) and the pedicle screw (14) within the receiving head (15).

25. The device as claimed in claim 23 or 24, **characterized in that** the circular collar (6; 23) has the diameter d, and the concave portion (10; 26) is of spherical design and has the diameter D, where D = d.

26. The device as claimed in claim 23 or 24, **characterized in that** the circular collar (6; 23) has the diameter d and the concave portion (10; 26) is of spherical design and has the diameter D, where D > d.

27. The device as claimed in claim 26, **characterized in that** the ratio d:D is between 0.5 and 1.0.

28. The device as claimed in claim 27, **characterized in that** the ratio d:D is between 0.85 and 0.95.

29. The device as claimed in claim 23 or 24, **characterized in that** the concave portion (10; 26) is designed in the manner of a spherical segment, where the spherical segment has a radius X, and X ≥ D/2.

30. The device as claimed in claim 29, **characterized in that** the ratio of D/2 to X is between 0.5 and 1.0.

31. The device as claimed in claim 30, **characterized in that** the ratio of D/2 to X is between 0.85 and 0.95.

32. The device as claimed in one of claims 23 through 31, **characterized in that** the rim of the collar (6; 23) is stepped and has at least one lower edge (28; 53; 54; 56).

33. The device as claimed in one of claims 23 through 32, **characterized in that** the collar (6) comprises a plurality of edges (53; 54; 56) concentric to the longitudinal axis (5; 24) of the screw, with diameters d > d₁ > d₂ decreasing toward the screw shank (4).

34. The device as claimed in claim 33, **characterized in that** the edges (53; 54; 56) are circular, and the diameters d; d1; d2 are dimensioned such that the edges (53; 54; 56) run on an imaginary convex surface (55) on the screw shank side and, in the concave portion (10; 26) of the bore (7; 17), can be made to bear on the wail (12; 27) of the bore (7; 17) at different angles between the longitudinal axis (5; 24) of the screw and the central axis (8; 16).

35. The device as claimed in claim 34, **characterized in that** the imaginary surface (55) is a spherical zone concentric to the central axis (8; 16) and with the radius Y.

36. The device as claimed in one of claims 23 or 25 through 35, **characterized in that** the fixation body (1) is a bone plate with at least one through-bore (7) for a bone screw (2), and the device additionally comprises a grub screw (45) with means for receiving a screwdriver, which can be screwed in an internal thread (46) introduced from the upper side (11) into the bore (7) and can be pressed against the screw head (22) upon tightening.

## Revendications

1. Vis d'ostéosynthèse (2;14) comportant un corps de vis (4;21) devant être ancré dans un os ou une partie d'os de manière concentrique à un axe longitudinal (5;24) de la vis ainsi qu'une tête de vis (3;22), la vis d'ostéosynthèse (2;14) présentant, entre la tête de vis (3;22) et le corps de vis (4;21) un renflement (6;23) en forme de disque, concentrique à l'axe longitudinal (5;24) de la vis, dont le diamètre est supérieur au diamètre du corps de vis (4;21),
le renflement (6;23) comprenant plusieurs arêtes (53;54;56) concentriques à l'axe longitudinal (5;24) de la vis,
**caractérisée en ce que**
A) les arêtes (53;54;56) décrivent chacune une ligne circulaire et présentent les diamètres d;d₁;d₂;dᵢ qui sont dimensionnés de manière à ce que les arêtes (53;54;56) s'inscrivent dans une surface imaginaire (55) convexe du côté du corps de vis, et
B) la surface imaginaire (55) est une zone sphérique concentrique par rapport à l'axe central (8;16) et présentant le rayon Y.

2. Vis d'ostéosynthèse (2;14) selon la revendication 1, **caractérisée en ce que** le renflement (6;23) présente deux arêtes (53;54;56) concentriques par rapport à l'axe longitudinal (5;24) de la vis.

3. Vis d'ostéosynthèse (2;14) selon la revendication 2, **caractérisée en ce que** les arêtes concentriques (53;54;56) du renflement (6;23) présentent un diamètre d > d₁ > d₂ > dᵢ allant en diminuant vers le corps de vis (4).

4. Vis d'ostéosynthèse (2) selon l'une des revendications 1 à 3, **caractérisée en ce que** la tête de vis (22) est de forme convexe.

5. Vis d'ostéosynthèse (2) selon la revendication 4, **caractérisée en ce que** la tête de vis (22) est de forme sphérique.

6. Vis d'ostéosynthèse (2) selon la revendication 5, **caractérisée en ce que** la tête de vis (22) est de forme hémisphérique, le zénith de la tête de vis (22) coupant l'axe longitudinal (24) de la vis à son extrémité.

7. Vis d'ostéosynthèse (2) selon l'une des revendications 1 à 6, **caractérisée en ce que** la tête de vis (3;22) est réalisé en une seul pièce avec le corps de vis (4;21).

8. Vis d'ostéosynthèse (2) selon l'une des revendications 1 à 6, **caractérisée en ce qu'**elle se compose de plusieurs pièces, dont au moins la tête de vis (3;22) et le corps de vis (4;21) sont des pièces séparées mais pouvant être assemblées de manière concentrique par rapport à l'axe longitudinal (5;24) de la vis.

9. Vis d'ostéosynthèse (2) selon la revendication 8, **caractérisée en ce que** la tête de vis (3;22) et le corps de vis (4;21) sont des pièces pouvant être assemblées de manière amovible,

10. Vis d'ostéosynthèse (2) selon la revendication 8, **caractérisée en ce que** la tête de vis (3;22), le renflement (6;23) et le corps de vis (4;21) sont des pièces séparées mais pouvant être assemblées de manière concentrique à l'axe longitudinal (5;24) de la vis.

11. Vis d'ostéosynthèse (2) selon l'une des revendications 8 à 10, **caractérisée en ce que** la tête de vis (3;22) est assemblée au corps de vis (4;21) au moyen d'un assemblage conique.

12. Vis d'ostéosynthèse (2) selon l'une des revendications 8 à 10, **caractérisée en ce que** la tête de vis (3;22) est assemblée au corps de vis (4;21) au moyen d'un assemblage vissé.

13. Vis d'ostéosynthèse (2) selon l'une des revendications 8 à 10, **caractérisée en ce que** la tête de vis (3;22) est assemblée au corps de vis (4;21) au moyen d'un verrouillage à baïonnette.

14. Vis d'ostéosynthèse (2) selon l'une des revendications 1 à 13, **caractérisée en ce que** le diamètre d du renflement (6;23) se situe entre 8 et 10 mm.

15. Vis d'ostéosynthèse (2) selon l'une des revendications 1 à 16, **caractérisée en ce que** le renflement (6;23) possède une épaisseur comprise entre 1 et 2 mm.

16. Vis d'ostéosynthèse (2) selon l'une des revendications 1 à 17, **caractérisée en ce que** le diamètre extérieur du corps de vis (4;21) se situe entre 5 et 6 mm.

17. Vis d'ostéosynthèse (2) selon l'une des revendications 1 à 15, **caractérisée en ce que** le diamètre d du renflement (6;23) se situe entre 4 et 6 mm.

18. Vis d'ostéosynthèse (2) selon la revendication 17, **caractérisée en ce que** le renflement (6;23) possède une épaisseur comprise entre 0,5 et 1 mm.

19. Vis d'ostéosynthèse (2) selon la revendication 18, **caractérisée en ce que** le diamètre extérieur du corps de vis (4;21) se situe entre 3 et 5 mm.

20. Vis d'ostéosynthèse (2) selon l'une des revendications 1 à 19, **caractérisée en ce qu'**elle sert à fixer des os ou des parties d'os dans un dispositif de fixation pour ostéosynthèse.

21. Vis d'ostéosynthèse (2) selon la revendication 20, **caractérisée en ce qu'**elle sert à fixer des os ou des parties d'os contre une plaque d'ostéosynthèse (1).

22. Vis d'ostéosynthèse (2) selon la revendication 20, **caractérisée en ce qu'**elle est une vis pédiculaire (14) et qu'elle sert à fixer des corps vertébraux dans un dispositif de fixation pour colonne vertébrale.

23. Dispositif permettant la fixation d'os à des fins d'ostéosynthèse, ledit dispositif comportant au moins une vis d'ostéosynthèse (2;14) selon l'une des revendications 1 à 22; et
A) au moins un corps de fixation (1;15) prismatique, cylindrique ou en forme de plaque présentant au moins un trou (7;17) avec un axe central (8;16) lequel permet de recevoir la vis d'ostéosynthèse (2;14), ainsi qu'une face inférieure (9;19) située du côté du corps de vis et une face supérieure (11;18) située du côté de la tête de vis,
B) le trou (7;17) comprenant une partie (10;26) allant en s'amincissant vers la face inférieure (9;19);
**caractérisé en ce que**
C) le diamètre d du renflement (6;23) est dimensionné de manière à ce qu'il soit possible, au niveau de la partie concave (10;26) du trou (7;17), de faire reposer le renflement (6;23) contre la paroi (12;27) dudit trou (7;17), et ce dans différents angles compris entre l'axe longitudinal (5;24) de la vis et l'axe central (8; 16).

24. Dispositif selon la revendication 23, **caractérisé en ce que**
A) la vis d'ostéosynthèse (2) est une vis pédiculaire (14) comportant une tête de vis convexe (22);
B) le corps de fixation (1) est une tête de réception (15) présentant l'axe central (16), laquelle sert à assembler un support longitudinal (13) avec la vis pédiculaire (14), et **en ce que** ladite tête de réception (15) présente en outre un canal (20) ouvert vers la face supérieure (18), qui s'étend transversalement à l'axe central (16) et permet de recevoir un support longitudinal (13); et
C) le dispositif comprend en outre des moyens de serrage (25) qui peuvent être assemblés de manière amovible, à partir de la face supérieure (18), avec la tête de réception (15) et qui servent à fixer un support longitudinal (13) et la vis pédiculaire (14) à l'intérieur de la tête de réception (15).

25. Dispositif selon la revendication 23 ou 24, **caractérisé en ce que** le renflement en forme de disque (6;23) présente le diamètre d, que la partie concave (10;26) est réalisée en forme de sphère et présente le diamètre D, D étant égal à d (D = d).

26. Dispositif selon la revendication 23 ou 24, **caractérisé en ce que** le renflement en forme de disque (6;23) présente le diamètre d, que la partie concave (10;26) est réalisée en forme de sphère et présente le diamètre D, D étant supérieur à d (D > d).

27. Dispositif selon la revendication 26, **caractérisé en ce que** le rapport d : D est compris entre 0,5 et 1,0.

28. Dispositif selon la revendication 27, **caractérisé en ce que** le rapport d : D est compris entre 0,85 et 0,95.

29. Dispositif selon la revendication 23 ou 24, **caractérisé en ce que** la partie concave (10;26) est réalisée sous forme de segment sphérique à deux bases, le segment sphérique à deux bases présentant un rayon X et X étant supérieur ou égal à D/2 (X ≥ D/2).

30. Dispositif selon la revendication 29, **caractérisé en ce que** le rapport de D/2 à X est compris entre 0,5 et 1,0.

31. Dispositif selon la revendication 30, **caractérisé en ce que** le rapport de D/2 à X est compris entre 0,85 et 0,95.

32. Dispositif selon l'une des revendications 23 à 31, **caractérisé en ce que** le bord du renflement (6;23) est réalisé sous forme d'arêtes et présente au moins une arête inférieure (28;53;54;56).

33. Dispositif selon l'une des revendications 23 à 33, **caractérisé en ce que** le renflement (6) comprend plusieurs arêtes (53;54;56) concentriques à l'axe longitudinal (5;24) de la vis et dont les diamètres d > d₁ > d₂ vont en s'amincissant vers le corps de vis (4).

34. Dispositif selon la revendication 33, **caractérisé en ce que** les arêtes (53;54;56) décrivent chacune une ligne circulaire et que les diamètres d;d₁;d₂ sont dimensionnés de manière à ce que les arêtes (53;54;56) s'inscrivent dans une surface imaginaire (55), convexe du côté du corps de vis et qu'il est possible, au niveau de la partie concave (10;26) du trou (7;17), de les faire reposer contre la paroi (12;27) dudit trou (7;17), et ce dans différents angles compris entre l'axe longitudinal (5;24) de la vis et l'axe central (8;16).

35. Dispositif selon la revendication 34, **caractérisé en ce que** la surface imaginaire (55) est une zone sphérique concentrique à l'axe central (8;16) et présentant le rayon Y.

36. Dispositif selon l'une des revendications 23 ou 25 à 35, **caractérisé en ce que** le corps de fixation (1) est une plaque d'ostéosynthèse comportant au moins un trou traversant (7) destiné à recevoir une vis d'ostéosynthèse (2) et que le dispositif comprend, en outre, un goujon fileté (45) avec des moyens permettant de recevoir un tournevis, ledit goujon fileté pouvant être vissé dans un filet intérieur (46) formé dans le trou (7) à partir de la face supérieure (11) et pouvant être pressé, lors du vissage, contre la tête de vis (22).
